(19) Europäisches Patentamt
European Patent Office
Office européen des brevets

(11) Publication number: **0 120 286**
**B1**

(12) **EUROPEAN PATENT SPECIFICATION**

(45) Date of publication of patent specification: **29.07.87**

(51) Int. Cl.$^4$: **A 01 N 25/02**

(21) Application number: **84101806.2**

(22) Date of filing: **21.02.84**

(54) **Pesticidal pour-on oil formulations.**

(30) Priority: **22.02.83 GB 8304927**

(43) Date of publication of application:
**03.10.84 Bulletin 84/40**

(45) Publication of the grant of the patent:
**29.07.87 Bulletin 87/31**

(84) Designated Contracting States:
**BE DE FR GB IT LU NL**

(56) References cited:
**EP-A-0 045 424**
**GB-A- 708 384**
**US-A-3 980 791**

(73) Proprietor: **THE WELLCOME FOUNDATION LIMITED**
**183-193 Euston Road**
**London NW1 2BP (GB)**

(72) Inventor: **Piercy, David William Thomas**
**17 South Park Road**
**Berkhamsted Herts. (GB)**

(74) Representative: **Sandmair, Kurt, Dr. et al**
**Patentanwälte Schwabe, Sandmair, Marx**
**Stuntzstrasse 16**
**D-8000 München 80 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 120 286 B1

**Description**

The present invention relates to pour-on formulations for localised topical applications comprising one or more ectoparasiticides in a glycol of glycerol ester of a $C_{8-10}$ fatty acid carrier and up to 10% weight to volume of additives conventionally used in pour-on formulations and to methods of controlling parasites of animals by administration of such formulations.

Formulations for localised topical application, such as pour-on or spot-on formulations are now well known in the art and are described, for example, in EP—A—0 045 424 and UK Patent Application 8134831 (Publication No. 2088212). These formulations are applied topically to a limited area of the animal's body surface and the ectoparasiticide is believed to migrate over the body surface to control ectoparasites distant from the points of application. The above mentioned EP—A—0 045 424 relates to pour-on formulations having an improved anti-tick effect containing 0,1—30 parts by weight of anti-tick agents, 10—80 parts by weight of one or more spreading oils, characterized by a surface tension at an air interface of $<30 \cdot 15^{5}$N/cm, 20—95 parts by weight of one or more solvents which are tolerated by the skin and 0—20 parts by weight of further additives.

Unfortunately, it has been discovered that a number of solvent systems described in the art provide formulations for localised topical application which cause irritancy or toxicity to the animal on administration. This is particularly the case with substances which are insoluble in a range of solvents, thereby restricting the choice of solvent that can be used to provide a pour-on formulation where the active substance is in solution.

It has now been found that a particular group of neutral oils has the combined properties of low irritancy and appropriate solvency and is particularly suitable for use in formulations for localised topical application. These neutral oils have the additional advantages of having low melting points and of not enhancing the transdermal penetration of the substance having ectoparasite activity.

Accordingly, the present invention provides a formulation comprising one or more ectoparasiticides in a glycol or glycerol ester of a $C_{8-10}$ fatty acid carrier and up to 10% weight to volume of additives conventionally used in pour-on formulations.

Suitably the ectoparasiticide is in a glycol diester or a glycerol triester of a $C_{8-10}$ fatty acid and preferably in a glycerol triester.

Suitably the carrier is fractionated coconut oil, also referred to as caprylic/capric triglyceride.

The formulations of the present invention are preferably solutions.

The present formulations may contain up to 10% w/v of additives conveniently used in pour-on formulations, for example spreading agents, adhesion promoters, surface active agents, stabilisers and colouring agents. Suitably the formulations of the present invention contain up to 10% w/v of such additives and preferably below 8% w/v of such additives.

In one preferred embodiment the formulations of the present invention will include less than 1% w/v of additives. Preferably the formulations will contain no additives, or colouring agents are the only additives included and these will be present at a level of 0.5% or less.

Suitable spreading agents are liquids which distribute themselves particularly readily on the skin. Dowanol DPM (dipropylene glycol mono methyl ether) is a particularly suitable spreading agent for inclusion with the formulations of the present invention. Isopropyl myristate is another commonly used spreading agent.

Adhesion promoters include carboxymethylcellulose, methylcellulose and other cellulose derivatives and starch derivatives, polyacrylates, alginates, gelatin, gum arabic, polyvinylpyrrolidone, polyvinyl alcohol, copolymers of methyl vinyl ether and maleic anhydride, polyethylene glycols, paraffins, oils, waxes and hydrogenated castor oil, colloidal silicic acid or mixtures of the substances mentioned.

The formulations of the present invention do not normally contain surface active agents; however these may be included if desired.

Surface-active agents (comprising emulsifiers and wetting agents) include:

1. anionic surface-active agents, such as Na lauryl sulphate, fatty alcohol ether-sulphates and monethanolamine salts of mono-/di-alkylpolyglycol ether orthophosphoric acid esters,

2. cationic surface-active agents, such as cetyltrimethylammonium chloride,

3. ampholytic surface-active agents, such as di-Na-N-lauryl-amino-dipropionate or lecithin, and

4. non-ionic surface-active agents, for example, polyoxyethylated castor oil, polyoxyethylated sorbitane monooleate, sorbitan monostearate, ethyl alcohol, glycerol monostearate, polyoxyethylene stearate and alkylphenol polyglycol ethers.

Stabilisers for preventing the chemical degradation which occurs in the case of some active compounds include, for example, antioxidants, such as tocophenols, butylhydroxyanisole and butylhydroxytoluene and scavengers such as epichlorohydrin. Colouring agents include conventional dyes which are soluble in the carrier of the present invention, such as Sudan Red or Oil Golden Yellow.

The ectoparasiticide incorporated within a formulation of the present invention may be active against one or more ectoparasite species including insects and acarines, including lice, ticks, keds, mites, fleas and itchmite.

Water insoluble ectoparasiticides agents are particularly suitable for inclusion in the present invention and include pyrethrins, pyrethroids, water-insoluble organo-phosphorus compounds, formamidines,

avermectins (or milbemycins) and mixtures thereof. Suitable milbemycins are disclosed in Australian published patent applications numbers 42309/78 and 42389/78. Where there are isomers of ectoparasiticides, both the ectoparasitically active isomers themselves and mixtures thereof with non-ectoparasitically active isomers are suitable for inclusion within the formulations of the present invention.

Preferred pyrethroids have the formula

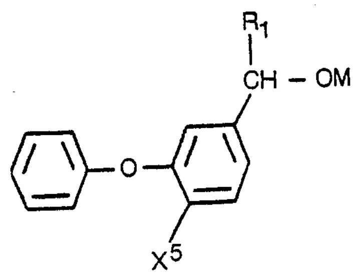

(1)

wherein M is

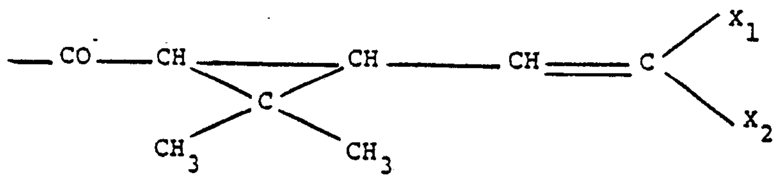

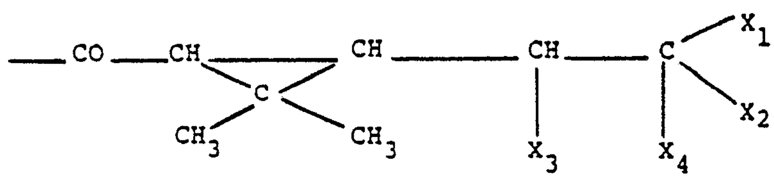

Or

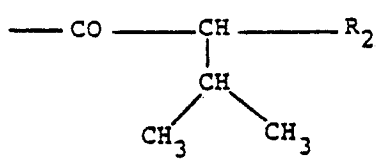

and wherein

$X_1$ to $X_4$ are independently selected from halo $C_1$—$C_4$ alkyl, halogen substituted $C_1$—$C_4$ alkyl, and halogen-substituted phenyl;

$X_5$ is —H or halo;

$R_1$ is —H or cyano; and

$R_2$ is halogen-substituted phenyl.

Particularly preferred compounds are presented in Tables I to III.

TABLE I

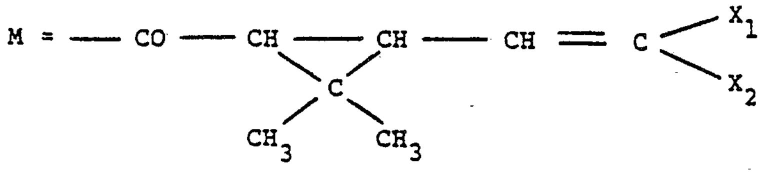

| No. | $X_1$ | $X_2$ | $X_3$ | $X_4$ | $X_5$ | $R_1$ | trivial name |
|---|---|---|---|---|---|---|---|
| 1 | CL | CL | — | — | H | H | permethrin |
| 2 | $CH_3$ | $CH_3$ | — | — | H | H | phenothrin |
| 3 | Br | Br | — | — | H | CN | deltamethrin |
| 4 | Cl | Cl | — | — | H | CN | cypermethrin |
| 5 | Cl | $CF_3$ | — | — | H | CN | cyhalothrin |
| 6 | Cl | 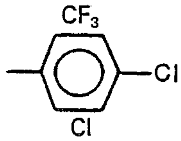 | — | — | F | CN | flumethrin |
| 7 | Cl | Cl | — | — | F | CN | cyfluthrin |
| 8 | $CH_3$ | $CH_3$ | — | — | H | CN | cyphenothrin |

TABLE II

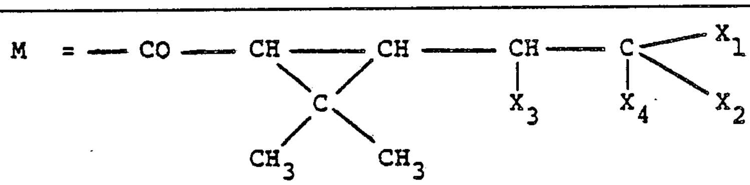

| No. | $X_1$ | $X_2$ | $X_3$ | $X_4$ | $X_5$ | $R_1$ | trivial name |
|---|---|---|---|---|---|---|---|
| 9 | Br | Br | Br | Br | H | CN | tralomethrin |
| 10 | Cl | Cl | Br | Br | H | CN | tralocythrin |

TABLE III

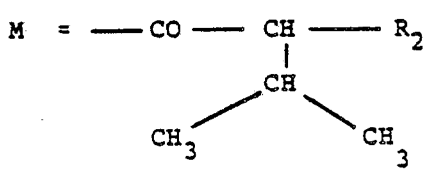

| No. | $R_2$ | $X_5$ | $R_1$ | trivial name |
|---|---|---|---|---|
| 11 | 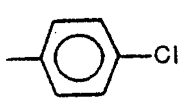 | H | CN | fenvalerate |

Deltamethrin, cypermethrin and Fastac are particularly suitable pyrethroids for inclusion within the formulations of the invention. Fastac is a 1:1 mixture of the IR-cis S and IS-cis R isomers of α-cyano-3-phenoxybenzyl 2,2-dimethyl-3-(2,2-dichlorovinyl)-cyclopropanecarboxylate. Deltamethrin is a particularly preferred pyrethroid. Deltamethrin's approved chemical name is (−)-α-cyano-3-phenoxybenyl (+)-cis-2,2-dimethyl-3-(2,2-dibromovinyl) cyclopropane-1-carboxylate.

Preferred water-insoluble organophosphorus compounds include the following:
O-(4-bromo-2,5-dichlorophenyl)-O,O-diethyl phosphorothioate (bromophos-ethyl)
2-chloro-1-(2,4-dichlorophenyl)-vinyl diethyl phosphate (chlorfenvinphos)
O,O-diethyl-O-(3,5,6-trichloro-2-pyridyl)phosphorothioate (chlorpyrifos)
O,O-diethyl-O-(3-chloro-4-methyl-7-coumarinyl)phosphorothioate (coumaphos);
O,O-diethyl-O-(2-isopropyl-6-methyl-pyrimidin-4-yl)phosphorothioate (diazonon®);
O-2,4-dichlorophenyl-O,O-diethylphosphorothioate (dichlofenthion)
2,3-p-dioxanedithiol S,S-bis (O,O,-diethylphosphorodithioate) (dioxathion);
O-ethyl-O-(quinolin-8-yl)-phenylphosphonothioate (oxinothiophos);
O,O,O,O-tetraethyl-S,S'-methylenedi(phosphorodithioate) (ethion)
O,O-dimethyl-O-2,4,5-trichlorophenylphosphorothioate (fenchlorphos);
O,O-dimethyl-O-(4-dimethylsulfamoylphenyl)phosphorothioate (famphur);
O,O-dimethyl-O-(4-nitro-m-tolyl)phosphorothioate (fenitrothion);
O,O-diethyl-α-cyanobenzylideneamino-oxyphosphonothioate (phoxim); and
(E)-O-2-isopropoxycarbonyl-1-methylvinyl-O-methyl-ethylphosphoramidothioate (propetamphos)

Preferred formamidines include water-insoluble compounds of the formula

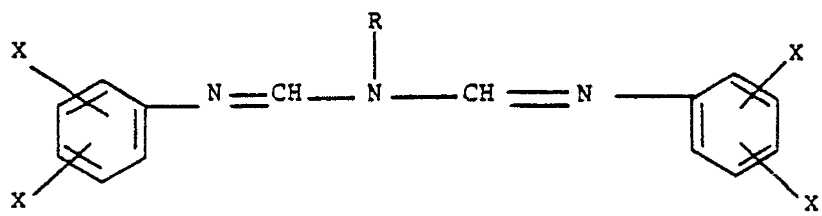

(II)

wherein R is hydrogen or $C_{1-6}$ alkyl, and each X is independently selected from hydrogen, $C_{1-6}$ alkyl and halo.

Particularly preferred formamidines include N,N-di-(2,4-xylyliminomethyl)-methylamine (called amitraz).

When a pyrethroid is included in a formulation of the present invention it will suitably be present at a concentration of 10% w/v or less. Deltamethrin will suitably be present at a concentration from 0.1 to 2.5%, preferably 0.5 to 2% and conveniently 1%.

Other active substances, as hereinbefore defined, will suitably be present at a concentration of less than 33%, suitably less than 15%, for example between 1 and 7.5%. Normally, there will be a maximum of three active substances in the formulation and preferably only one. Preferred mixtures of active substances include a pyrethroid with a second pyrethroid, with a water insoluble organophosphorus compound or with a formamidine.

The invention in a second aspect provides a method of controlling external parasites which comprises making a localised external application of a formulation as hereinbefore defined to an animal. External parasites include those of the Classes Arachnida (especially acarines) and Insecta (especially Phthiraptera and Diptera). External parasites of particular commercial significance include:

*Sheep*
*Melophagus ovinus* — ked
*Damalinia ovis* — biting louse
*Linognathus ovillus* — sucking louse
*L. Pedalis* — sucking louse

*Goats*
*Damalinia caprae* — biting louse
*D. limbata* — biting louse
*D. crassipes* — biting louse
*Linognathus stenopsis* — sucking louse

*Cattle*
a) Lice
*Damalinia bovis* — biting louse
*Linognathus vituli* — sucking louse
*Haematopinus eurysternus* — sucking louse
*Solenopotes capillatus* — sucking louse

b) Flies

*Musca domestica*
*M. autumnalis*
*Stomoxys calcitrans*
*Lyperosia irritans*
*Haematobia thirouxi potans*
*H. exigua*
*Simulium* spp.

c) Ticks

*Boophilus microplus*
*B. decoloratus*
*Rhipicephalus appendiculatus*
*R. evertsi*
*Amblyomma hebraeum*
*A. variegatum*
*Hyalomma rufipes*
*H. truncatum*
*Haemaphysalis longicornis*

*Pigs*

*Sarcoptes scabiei var suis* — mange
*Haematopinus suis* — sucking louse

*Dog*

*Linognathus setosus* — sucking louse
*Trichodectes canis* — biting louse
*Ctenocephalides canis* — flea
*Rhipicephalus sanguineus* — tick
*Haemaphysalis leachii* — tick

The animal is preferably a mammal, and may be selected from cattle, goats, pigs, horses, deer, sheep and domestic pets such as cats and dogs. The animal may also be a bird, e.g. selected from ducks, chickens and geese. Suitably the animal is selected from cattle, sheep and pigs. Preferably the formulations of the present invention will be used to treat lice and keds on sheep and goats and lice, flies and ticks on cattle.

The pour-on formulation may be applied to the animal by any conventional method for the localised application of formulations, for example by wiping an impregnated material over a small area of the animal's body or by the use of commercially available applicators, such as the Clout Backliner Applicator (Registered Trade mark) or a pump dispenser or by the apparatus described in Australian Patent No. 494198. Generally, the pour-on formulation is applied by pouring in one or several lines or in a spot on the back or shoulder of the animal. Alternatively, it may be applied by means of a localised spray.

It is a particular advantage of the use of pour-on formulations that only small volumes of the formulation need to be applied. Depending on the size of the animal, the volume applied will generally lie in the range 2—30 ml, and suitably 5—10 ml for larger mammals. The amount of active substance, as hereinbefore defined, administered to an animal will depend on the size of the animal, the amount can be between 10 mg and 10 g but will normally be between 10 mg and 1g. Preferably 50 to 100 mg of active substance will be applied to a sheep and 100 to 200 mg of active substance will be applied to a cow.

The formulations of the present invention will be prepared by standard techniques i.e. in the case where the formulation is a solution by bringing the ectoparasiticide into contact with the carrier and then gently heating and stirring until dissolved if necessary. If the ectoparasiticide is insoluble in or immiscible with the carrier or is water-soluble, then a suspension or emulsion may be prepared by standard techniques. To prepare a suspension the ectoparasiticide may be ground in the solvent to the required particle size and the remaining excipients added with stirring until the final product is of uniform consistency; generally heating is not necessary. To prepare an emulsion, the ectoparasiticide is dissolved in the carrier or water (whichever is appropriate) together with a suitable emulsifier and the whole homogenised by conventional means. Emulsions and suspensions are not preferred formulations of the present invention.

Preferred formulations will now be described by way of example only as follows:

### General Procedure for Preparing Formulations

General Preparation:

In the case of a solid the active ingredient (for example deltamethrin) is added to the solvent with stirring. Gentle heat is applied, where necessary, and stirring is continued until all the solid has dissolved and a homogenous mixture is obtained. Auxiliaries to be included in the formulation may be either mixed

with the active ingredient before addition of the solvent or added to the homogenous mixture of active ingredient and solvent.

Combinations of active ingredients will be prepared in the same manner as formulations of single active ingredients.

In the case where the active ingredient(s) is a liquid (e.g. supona), then the preparation is prepared by mixing the two (or more) liquids together until the product is homogenous. Heat will not generally be necessary.

Example 1

Solutions of deltamethrin in fractionated coconut oil (Miglyol 812 from Dynamit Nobel Chemicals) were prepared by the standard technique described before.

| | | |
|---|---|---|
| a) | Deltamethrin | 1 g |
| | Miglyol 812 | To 100 ml |
| b) | Deltamethrin | 1 ml |
| | Dowanol DPM | 10 g |
| | Miglyol 812 | To 100 ml |

Sudan Red 0.02 g was included in each formulation.
Miglyol is a Registered Trade Mark.

Example 2

A solution of amitraz in fractionated coconut oil was prepared as described before.

| | |
|---|---|
| Amitraz | 5 g |
| Miglyol 812 | To 100 ml |

Sudan Red 0.02 g was included in each formulation.

Example 3

Solutions of other ectoparasiticides were prepared in Miglyol 812 in a similar manner.

| | | |
|---|---|---|
| 1) | PERMETHRIN | 5 g |
| | MIGLYOL 812 | To 100 ml |
| 2) | CYPERMETHRIN | 2.5 g |
| | MIGLYOL 812 | To 100 ml |
| 3) | ETHION | 10 g |
| | MIGLYOL 812 | To 100 ml |
| 4) | DELNAV (Dioxathion) | 10 g |
| | MIGLYOL 812 | To 100 ml |
| 5) | PROLATE | 5 g |
| | MIGLYOL 812 | To 100 ml |
| 6) | DIELDRIN | 1 g |
| | MIGLYOL 812 | To 100 ml |
| 7) | DIAZINON® | 7 g |
| | MIGLYOL 812 | To 100 ml |
| 8) | SUPONA | 7 g |
| | MIGLYOL 812 | To 100 ml |
| 9) | PERMETHRIN | 5 g |
| | ETHION | 10 g |
| | MIGLYOL | To 100 ml |
| 10) | DELTAMETHRIN | 1 g |
| | ETHION | 5 g |
| | MIGLYOL | To 100 ml |

Example 4

Solutions of ectoparasiticides were prepared in Miglyol 840 in a similar manner.

| | | |
|---|---|---|
| 1) | DELTAMETHRIN | 1 g |
| | MIGLYOL 840 | To 100 ml |
| 2) | DELTAMETHRIN | 1 g |
| | BUTYLATED HYDROXYANISOLE | 0.5 g |
| | MIGLYOL 840 | To 100 ml |
| 3) | AMITRAZ | 5 g |
| | STABAXOL 1® | 1 g |
| | MIGLYOL 840 | To 100 ml |

A field trial to determine the efficacy of 1% Deltamethrin in Miglyol 812, applied as a 'spot-on', for the treatment of a lice infestation on pigs

Introduction

The objective of the study was to determine the efficacy of 1% deltamethrin in Miglyol 812 for the treatment of a lice infestation on pigs.

Animals

Two hundred and sixty 'Fastback Hybrid' pigs comprising 37 gilts, 9 boars and 214 sows.

Materials

| | |
|---|---|
| Deltamethrin | 10.7 g/kg |
| Miglyol 812 | 989.3 g/kg |
| | 1000.0 |

Method

Of the 260 gilts, sows and boars on the farm a total of 239 were examined to determine whether they were infested with the pig louse, *Haematopinus suis.* Records were kept of the ear tag number of the pigs and of the level of infestation. This was classified subjectively as:

| | |
|---|---|
| + | Light |
| ++ | Moderate |
| +++ | Heavy |

All of the 260 gilts, sows and boars were treated with 5 ml deltamethrin 1% in Miglyol 812. This was applied topically to the midline of the shoulders using a Clout Backliner (Wellcome Australia). (Clout is a registered trade mark).

Examination and treatment were performed on April 12 1983. Included in the pigs examined and treated were 44 sows housed in farrowing accommodation. Most of these were suckling piglets and the remainder were due to farrow within a few days after treatment. The suckling piglets were examined for lice infestation but were not treated.

The treated pigs were examined immediately after application of the deltamethrin for any signs of clinical toxicity. The farmer was asked to report any adverse effects.

All 260 gilts, sows and boars were re-examined on April 22nd 1983 and May 24th 1983.

Results

Details of the animals examined and treated, and the level of lice infestation, are appended as Table 1.

Of the 239 pigs examined on April 12th 1983, 235 were lightly to moderately infested with *Haematopinus suis.* No lice were found on four gilts.

No signs of clinical toxicity were observed after treatment and at the subsequent examination of April 22nd the farmer reported no adverse effects. He noted that within 24 hours of treatment lice were visible at the ends of hairs. He also remarked that it was apparent 24 hours after treatment where the formulation had spread, but by 48 hours there was no indication of where the formulation had been applied.

At this examination, 10 days after treatment, no live lice were observed on any of the 260 pigs examined. No live lice were found on the sucking piglets.

Similarly, no live lice were found on the treated pigs nor on the untreated piglets at the examination on May 24, 42 days after treatment.

Discussion

In this trial 5 ml of deltamethrin 1% in Miglyol 812 proved very successful in controlling a light to moderate infestation of *Haematopinus suis* infestation on pigs. No live lice were found at examinations 10 days and 42 days after treatment. Under normal circumstances, on untreated animals, a 42 day period should have been sufficient for viable eggs to hatch and for ovigerous females to be apparent.

After treatment of suckling sows no live lice were found on their untreated piglets which implies that the piglets were protected from lice infestation by treatment of their dams.

No evidence was seen of skin irritation immediately after treatment nor of subsequent skin damage.

TABLE 4

Results of pre-treatment examination on April 12th 1983

Middle House

| Pen Number | 1 | 2 | 3 | 4 | 5 | 6 | 7 | 8 | 9 | 10 |
|---|---|---|---|---|---|---|---|---|---|---|
| Sow Number | 436<br>401<br>529 | 3 pigs<br>NE | 3 pigs<br>NE | 632<br>618<br>440 | 3 pigs<br>NE | 3 pigs<br>NE | 637<br>656<br>633 | 640<br>638<br>643 | 549<br>600<br>346 | 540<br>(boar) |
| Infestation | ++ | NE | NE | ++ | NE | NE | ++ | ++ | ++ | ++ |

Far House

| Pen Number | 1 | 2 | 3 | 4 | 5 | 6 | 7 | 8 | 9 | 10 |
|---|---|---|---|---|---|---|---|---|---|---|
| Sow Number | 3 pigs<br>NE | 3 pigs<br>NE | 3 pigs<br>NE | 506<br>588<br>530 | 481<br>454<br>550 | 617<br>456<br>595 | 505<br>587<br>511 | 474<br>400<br>429 | 602<br>603<br>549 | 652<br>(boar) |
| Infestation | NE | NE | NE | ++ | ++ | ++ | ++ | ++ | ++ | ++ |

Near House (Gilts)

| Pen Number | 1 | 2 | 3 | 4 | 5 | 6 | 7 | 8 | 9 | 10 |
|---|---|---|---|---|---|---|---|---|---|---|
| Sow Number | 4 gilts | 4 gilts | 4 gilts | 4 gilts | Boar | 4 gilts | 4 gilts | 4 gilts | 657<br>660<br>663<br>670 | 648<br>646<br>655<br>673<br>674 |
| Infestation | + | + | + | + | + | + | − | + | + | + |

TABLE 4 (cont)

| House | | 1 | 2 | 3 | 4 | 5 | 6 | 7 | 8 |
|---|---|---|---|---|---|---|---|---|---|
| House 1 | Pen No. | 1 | 2 | 3 | 4 | 5 | 6 | 7 | 8 |
| | Sow Number | 488<br>616<br>418 | 608<br>614<br>641 | 412<br>515<br>636 | 623<br>405<br>642 | 574<br>573<br>650 | Empty | 332<br>541 | 506<br>(boar) |
| | Infestation | + | + | + | + | + | | + | + |
| House 2 | Pen No. | 1 | 2 | 3 | 4 | 5 | 6 | 7 | 8 |
| | Sow Number | 368<br>414<br>458 | 590<br>324<br>89 | 547<br>559<br>516 | 622<br>586<br>363 | 528<br>634<br>635 | 386<br>593<br>565 | 664 | 654<br>(boar) |
| | Infestation | ++ | ++ | ++ | ++ | ++ | ++ | ++ | ++ |
| House 3 | Pen No. | 1 | 2 | 3 | 4 | 5 | 6 | 7 | 8 |
| | Sow Number | 499<br>570<br>453 | 318<br>592 | 542<br>348<br>441 | 349<br>553<br>373 | 626<br>627<br>624 | 671<br>507<br>628 | 546<br>571<br>649 | 414<br>(boar) |
| | Infestation | + | ++ | + | ++ | ++ | ++ | ++ | + |
| House 4 | Pen No. | 1 | 2 | 3 | 4 | 5 | 6 | 7 | 8 |
| | Sow Number | 374<br>484<br>579 | 477<br>271<br>639 | 563<br>499<br>489 | 426<br>452<br>620 | 330<br>577<br>464 | 514<br>473<br>598 | 443<br>665<br>630 | 123<br>(boar) |
| | Infestation | + | + | ++ | ++ | ++ | ++ | ++ | ++ |
| House 5 | Pen No. | 1 | 2 | 3 | 4 | 5 | 6 | 7 | 8 |
| | Sow Number | 585<br>587<br>502 | 416<br>625<br>619 | 645<br>414<br>291 | 382<br>581<br>493 | 439<br>334<br>522 | 647<br>402<br>? | 557<br>672<br>582 | 252<br>(boar) |
| | Infestation | + | + | + | ++ | + | ++ | ++ | + |
| House 6 | Pen No. | 1 | 2 | 3 | 4 | 5 | 6 | 7 | 8 |
| | Sow Number | 647<br>527<br>534 | 547<br>408<br>538 | 97<br>520<br>292 | 532<br>536<br>539 | 435<br>606<br>609 | 298<br>576<br>479 | Empty | 682<br>(boar) |
| | Infestation | ++ | + | + | ++ | ++ | ++ | | ++ |

(continuing from table 4)

Farrowing House

Sow numbers:

| | | | |
|---|---|---|---|
| 406 | 535 | 457) | |
| 653 | 425 | 470) | |
| 556 | 439 | 433) | |
| | | ) | |
| 380 | 494 | 402) | |
| 444 | 317 | 533) | |
| | | ) | |
| | 377 | 519) | |
| 544 | 320 | 562) | All |
| | | ) | + infestation |
| 568 | 543 | 285) | |
| 651 | 652 | 654) | |
| 363 | 578 | 460) | |
| 601 | 445 | 487) | |
| | | ) | |
| 479 | 458 | 439) | |
| 460 | 436 | 407) | |
| 555 | 605 | 599) | |
| 510 | 561 | 506) | |

\+ = Slight infestation
++ = Moderate infestation
NE = Not examined
— = No live lice found

## Example 6

A field trial to assess the efficacy of a formulation containing 1.0% Deltamethrin against cattle lice

Introduction

Housed cattle with infestations of lice during the winter time often require treatment. The objective of this trial was to assess the efficacy of a 1.0% deltamethrin formulation when applied to lice infested cattle.

Materials

Formulation containing 1.0% deltamethrin based on Miglyol 812 as solvent.

Lice infested cattle in the West of Scotland. Details of farms A, B and C and the cattle are given in Table 5.

Method

The cattle at each farm were examined for the presence of lice. The lice were identified and the severity of the infestation was assessed and classified on a subjective basis as light, moderate or heavy. Each animal selected for treatment was identified by its ear tag number on farms A and B. On farm C, 7 out of 37 in the group were identified. All animals within a group were treated. After treatment there was no contact between treated and un-treated animals on the farm.

All the animals within a group were each treated with 10 ml of 1.0% deltamethrin formulation applied topically to the mid-point of the shoulders, using a Wellcome 20 mL Automatic Drencher.

Treated animals were examined for any signs of dermal irritation at the time of application and again 24 hours later.

The identified animals were re-examined thoroughly 21 days and 42 days after treatment for the presence of lice.

Results

The results are presented in Table 6.

Discussion

Out of 66 animals treated, 36 were examined of which 17 were infested with *Damalinia bovis,* 9 with *Linognathus vituli,* 14 with *Haematopinus eurysternus* and one with *Solenopotes capillatus*; some animals were infested with more than one species. On examination 3 weeks after treatment no live *D. bovis, H. eurysternus* or *S. capillatus* were detected; *L. vituli* were absent from the animals except for a few lice

present in the hollow of the hock joints of 3 animals and two *L. vituli* were present on the rump of one animal which had not been infested at the time of treatment.

On examination 6 weeks after treatment no live *D. bovis, H. eurysternus* or *S. capillatus* were detected; a few *L. vituli* were present in the hollow of the hock joints of two animals and one *L. vituli* was present on the rump of another, but otherwise *L. vituli* were not detected. Absence of live lice on examination at 6 weeks after treatment indicates that the infestation has been eradicated, as this allows sufficient time for any lice surviving the treatment to have reproduced and completed their life cycle.

Conclusion

Application of one treatment of 10 ml of the 1.0% deltamethrin formulation to lice infested cattle will give complete control of *Damalinia bovis, Haematopinus eurysternus, Solenopotes capillatus,* and virtually complete control of *Linognathus vituli.*

TABLE 5

Details of Trial Sites

| | | |
|---|---|---|
| Farm A | | |
| | Animals | 9 Ayrshire X Friesian. 6—12 months old. |
| | Housing | Tied in stalls. |
| Farm B | | |
| | Animals | 20 Hereford X Friesian. 6—12 months old. |
| | Housing | Tied in stalls. |
| Farm C | | |
| | Animals | 37 (7 identified) Friesian. 2—3 months old. |
| | Housing | Loose in pens. |

TABLE 6

Results of examination for lice
Treated: 18.1.83

Farm A.

| Animal No. | Pre-treatment 18.1.83 | | | | Post-treatment 8.2.83 | | | | Post-treatment 1.3.83 | | | |
|---|---|---|---|---|---|---|---|---|---|---|---|---|
| | D | L | H | S | D | L | H | S | D | L | H | S |
| 262 | ++ | – | – | – | – | +[1] | – | – | +[2] | – | – | – |
| 286 | ++ | – | – | – | – | – | – | – | – | – | – | – |
| 288 | + | – | – | – | – | – | – | – | – | – | – | – |
| BW | + | – | – | – | – | – | – | – | – | – | – | – |
| 211 | ++ | ++ | – | – | – | – | – | – | – | – | – | – |
| 27[illegible] | + | – | – | – | – | – | – | – | – | – | – | – |
| BW | + | – | – | – | – | – | – | – | – | – | – | – |
| 230 | + | – | – | – | – | – | – | – | – | – | – | – |
| 266 | ++ | – | – | – | – | – | – | – | – | – | – | – |

D = Damalinia bovis
L = Linognathus vituli
H = Haematopinus eurysternus
S = Solenopotes capillatus

+[1] = 2 live L.V. on rump
+[2] = 1 live L.V. on rump
– = No live lice found
\+ = Light infestation
++ = Moderate infestation
+++ = Heavy infestation

TABLE 6 (cont)

Results of examination for lice
Treated: 18.1.83

Farm B.

| Animal No. | Pre-treatment 18.1.83 | | | | Post-treatment 8.2.83 | | | | Post-treatment 1.3.83 | | | |
|---|---|---|---|---|---|---|---|---|---|---|---|---|
| | D | L | H | S | D | L | H | S | D | L | H | S |
| R174 | – | – | ++ | – | – | – | – | – | – | – | – | – |
| F927 | – | – | +++ | – | – | – | – | – | – | – | – | – |
| 939 | – | – | ++ | – | – | – | – | – | – | – | – | – |
| 1330 | – | – | + | + | – | – | – | – | – | – | – | – |
| P597 | ++ | – | – | – | – | – | – | – | – | – | – | – |
| HX1 | + | – | – | – | – | – | – | – | – | – | – | – |
| HX2 | ++ | – | – | – | – | – | – | – | – | – | – | – |
| 656 | + | – | – | – | – | – | – | – | – | – | – | – |
| HX3 | + | – | – | – | – | – | – | – | – | – | – | – |
| HX4 | – | – | +++ | – | – | – | – | – | – | – | – | – |
| HX5 | – | – | +++ | – | – | – | – | – | – | – | – | – |
| HX6 | – | + | – | – | – | – | – | – | – | – | – | – |
| A1160 | – | – | + | – | – | – | – | – | – | – | – | – |
| B47 | – | – | + | – | – | – | – | – | – | – | – | – |
| W967 | – | – | +++ | – | – | – | – | – | – | – | – | – |
| 303 | – | – | ++ | – | – | – | – | – | – | – | – | – |
| HX7 | – | – | +++ | – | – | – | – | – | – | – | – | – |
| B60 | – | – | + | – | – | – | – | – | – | – | – | – |
| A373 | – | – | ++ | – | – | – | – | – | – | – | – | – |
| HX8 | – | – | + | – | – | – | – | – | – | – | – | – |

D = Damalinia bovis
L = Linognathus vituli
H = Haematopinus eurysternus
S = Solenopotes capillatus

– = No live lice found
+ = Light infestation
++ = Moderate infestation
+++ = Heavy infestation

TABLE 6 (cont)

Results of examination for lice
Treated: 18.1.83

Farm C.

| Animal No. | Pre-treatment 18.1.83 D | L | H | S | Post-treatment 8.2.83 D | L | H | S | 1.3.83 D | L | H | S |
|---|---|---|---|---|---|---|---|---|---|---|---|---|
| 50 | – | + | – | – | – | * | – | – | – | – | – | – |
| 53 | – | + | – | – | – | * | – | – | – | * | – | – |
| 45 | – | ++ | – | – | – | * | – | – | – | – | – | – |
| 28 | – | +++ | – | – | – | – | – | – | – | * | – | – |
| 32 | +++ | ++ | – | – | – | – | – | – | – | – | – | – |
| 43 | +++ | + | – | – | – | – | – | – | – | – | – | – |
| 33 | +++ | + | – | – | – | – | – | – | – | – | – | – |

L. vituli were present in hollow of hock joints of all animals at the time of treatment.

D = Damalinia bovis
L = Linognathus vituli
H = Haematopinus eurysternus
S = Solenopotes capillatus

– = No live lice found
+ = Light infestation
++ = Moderate infestation
+++ = Heavy infestation
* = A few L.A. in hollow of hock joint.

Example 7

No discomfort seen in sheep after the application of Deltamethrin spot-on formulations

Materials

A formulation containing 2% w/v deltamethrin was evaluated. Details are given in Table 7.

5 groups of 4 animals each, details of which are given in Table 8.

Method

5 ml of 15 ml of each formulation was applied by polythene disposable syringes, spotted (5 ml) or poured (15 ml), along the midline of the back from the shoulders in each animal.

Each animal was observed independently by veterinary surgeons at intervals up to 48 hours after treatment for the following signs of discomfort:—

head shaking/movements
muscle fasciculation
dermal irritancy/rubbing
foot stamping

The severity of each sign was scored as follows:—

0 = no reaction/abnormal behaviour detected
1 = slight reaction
2 = moderate reaction
3 = marked reaction
4 = very marked reaction

The observers were unaware which formulation had been applied to each group.

At each observation the total score for each sign of discomfort was calculated as a percentage of the maximum possible. The percentages for each sign were added to give the total score for the group at each observation.

For example:—

| Animal Number | Head Shaking | Muscle fasciculation | Dermal irritancy | Foot stamping |
|---|---|---|---|---|
| A | 1 | 1 | 1 | 0 |
| B | 2 | 1 | 1 | 1 |
| C | 1 | 1 | 1 | 0 |
| | — | — | — | — |
| Total | 4 | 3 | 3 | 1 |
| % Maximum (12) | 33 | 25 | 25 | 8 |

Total score = 91

Results

The average scores for each group are recorded in Table 9.

Discussion

No signs of irritancy caused by spot-on application were seen in any animal. It appears sheep are not irritated by deltamethrin applied to the skin in doses three times the recommended application rate. It is concluded, within the limitations of this small study, that use of either formulation in sheep should cause no irritancy problems in the field.

TABLE 7

Details of Formulations

| | | | |
|---|---|---|---|
| Group 1 | Formulation 1 | % w/w | |
| | Miglyol 812 | 100.00 | Ex stock |
| | | 100.00 | |
| Groups 2 and 3<br>4 and 5 | Formulation 2 | % w/w | |
| | 2% Deltamethrin | 2.13 | |
| | Miglyol 812 | 97.87 | |
| | | 100.00 | |

TABLE 8

Details of Animal Groups

| Group No. | Number of animals | Wt. Range (kg) | Age (months) | Breed | Wool coat |
|---|---|---|---|---|---|
| 1 | 4 | 86—99 | 12 | Cheviot | shorn |
| 2 & 4 | 4 | 77—84 | 12 | Cheviot | long |
| 3 & 5 | 4 | 90—97 | 12 | Cheviot | shorn |

TABLE 9

Total Scores for Observations of Groups following Treatment

| Group No. | Formulation | ml | Observation Times (Hours) 0 | 1 | 3 | 6 | 24 | 36 |
|---|---|---|---|---|---|---|---|---|
| 1 | Formulation 1 | 5 | 0 | 0 | 0 | 0 | 0 | 0 |
| 2 | Formulation 2 | 5 | 0 | 0 | 0 | 0 | 0 | 0 |
| 3 | Formulation 2 | 5 | 0 | 0 | 0 | 0 | 0 | 0 |
| 4 | Formulation 2 | 15 | 0 | 0 | 0 | 0 | 0 | 0 |
| 5 | Formulation 2 | 15 | 0 | 0 | 0 | 0 | 0 | 0 |

The score given is the average of assessments made by 4 observers.

## Example 8

Effect of formulation on the efficacy of deltamethrin spot-on formulations against sheep keds and lice.

### Introduction

The objective of this trial was to assess the biological efficacy of two different deltamethrin spot-on formulations.

### Materials and Methods

i) Spot-ons

Two spot-on formulations, were used:

i) a 2% w/v aqueous suspension of deltamethrin

ii) a 1% w/v solution deltamethrin in Miglyol 812

ii) Sheep

Two test groups, each comprising 5 Welsh Mountain lambs, aged approximately 10 months, were used. The lambs were in full fleece (15 cm) and were infested with *Melophagus ovinus, Damalinia ovis* and *Linognathus ovillus.*

iii) Method of Application

5 ml of each formulation was applied to the skin of each sheep on either 18th or 25th February, 1983. The fleece was parted at the point of the shoulders or on the rump (mid-line in the sacro-lumbar region) before application.

Details of the treatments are given below:

| Group | Spot-on | Application Site | Treated |
|---|---|---|---|
| 1 | 2% aqueous suspension of deltamethrin | Shoulders | 25.2.83 |
| 2 | 1% solution of deltamethrin in Migylol 812 | Shoulders | 18.2.83 |

On both days the weather was dry and cool (5.5 or 7.8°C, respectively).

iv) Assessment of Parasite Burdens

The parasite burden of the test groups were assessed before and after treatment by standard techniques. The test groups were isolated for the period of the trial (11 weeks). No re-infestation techniques were employed.

### Results/Discussion

Keds

Full detail of the ked counts were given in Table 10. They are summarised in Table 11.

The Miglyol 812 formulation virtually eradicated all keds by week 7. The aqueous suspension formulation was longer taking effect.

TABLE 10
Total number of keds found at each examination.

| Group | Material used | Keds | Pre-treat count | Weeks 1 | 3 | 5 | 7 | 9 | 11 |
|---|---|---|---|---|---|---|---|---|---|
| 1 | 2% w/v aqueous suspension of Deltamethrin (Point of shoulders) | Fed Alive | 427 | 166 | 52 | 59 | 16 | 2 | 0 |
| | | Fed Affected | 0 | 14 | 1 | 3 | 0 | 0 | 0 |
| | | Fed Dead | 0 | 116 | 36 | 56 | 10 | 9 | 5 |
| | | Unfed Alive | 0 | 5 | 5 | 2 | 1 | 0 | 0 |
| | | Unfed Affected | 0 | 1 | 0 | 0 | 0 | 0 | 0 |
| | | Unfed Dead | 0 | 5 | 0 | 8 | 5 | 4 | 1 |
| | | Total | 427 | 307 | 94 | 128 | 32 | 15 | 6 |
| 2 | 1% w/v solution of Deltamethrin in Miglyol 812 (Point of shoulders) | Fed Alive | 286 | 70 | 12 | 5 | 0 | 1 | 0 |
| | | Fed Affected | 0 | 17 | 1 | 0 | 0 | 0 | 0 |
| | | Fed Dead | 0 | 10 | 50 | 20 | 15 | 11 | 1 |
| | | Unfed Alive | 0 | 8 | 3 | 1 | 0 | 1 | 0 |
| | | Unfed Affected | 0 | 2 | 3 | 0 | 0 | 0 | 0 |
| | | Unfed Dead | 0 | 6 | 11 | 15 | 10 | 3 | 1 |
| | | Total | 286 | 113 | 80 | 41 | 25 | 16 | 2 |

TABLE 11
% Survival of Original Infestation — weeks after treatment
(Figures in brackets represent group mean % survival of adult keds)

| Group | Material used | 5 ml applied on: | 1 | 3 | 5 | 7 | 9 | 11 |
|---|---|---|---|---|---|---|---|---|
| 1 | 2% w/v Deltamethrin (Suspension conc.) | Point of shoulders | 38.9 (58.4) | 12.2 (55.2) | 13.8 (52.3) | 3.8 (43.3) | 0.5 (13.3) | 0 (0) |
| 2 | 1% w/v Deltamethrin (Miglyol 812) | Point of shoulders | 24.5 (34.4) | 4.2 (14.3) | 1.8 (9.4) | 0 (0) | 0.3* (20.0) | 0 (0) |

* One live ked only

Lice

The results obtained against both species of lice are given in Table 12.

TABLE 12
Louse control. No. of sheep bearing live lice — weeks

| Group | Material used | 5 ml applied on: | Species | 1 | 3 | 5 | 7 | 9 | 11 |
|---|---|---|---|---|---|---|---|---|---|
| 1 | 2% aqueous suspension of Deltamethrin | Point of shoulders | *D. ovis* | 5 | 5 | 5 | 2 | 3 | 0 |
| | | | *L. ovillus* | 5 | 3 | 3 | 2 | 3 | 2 |
| 5 | 1% w/v solution Deltamethrin in Miglyol 812 | Point of shoulders | *D. ovis* | 5 | 1 | 0 | 0 | 0 | 0 |
| | | | *L. ovillus* | 4 | 2 | 3 | 2 | 1 | 0 |

The 1% w/v solution of deltamethrin in Miglyol 812 eradicated the *D. ovis* population by 5 weeks after application. The 2% w/v aqueous suspension of deltamethrin took the full 11-week trial period to clear this species.

The 1% w/v solution deltamethrin in Miglyol 812 was the only material to eradicate the less-impnerial to eradicate the less-important species, *L. ovillus.*

## Claims

1. Pour-on formulations comprising one or more ectoparasiticides in a glycol or glycerol ester of a $C_{8-10}$ fatty acid carrier and up to 10% weight to volume of additives conventionally used in pour-on formulations.

2. Pour-on formulation according to claim 1 wherein the carrier is a glycol diester or a glycerol triester of a $C_{8-10}$ fatty acid.

3. Pour-on formulation according to claim 2 wherein the carrier is fractioned coconut oil.

4. Pour-on formulation according to any of claims 1 to 3 wherein the ectoparasiticide is in solution.

5. Pour-on formulation according to any of claims 1 to 4 which contains a spreading agent.

6. Pour-on formulation according to any of claims 1 to 5 which contains less than 1% weight to volume of additives.

7. Pour-on formulation according to claim 6 which contains one or more stabilisers and/or colouring agents.

8. Pour-on formulation according to claim 1 wherein the ectoparasiticide is selected from the pyrethrins, pyrethroids, water-insoluble organophosphorus compounds, formamidines, avermectins and mixtures thereof.

9. A formulation according to claim 8 wherein the ectoparasiticide is a pyrethroid of the formula (I):

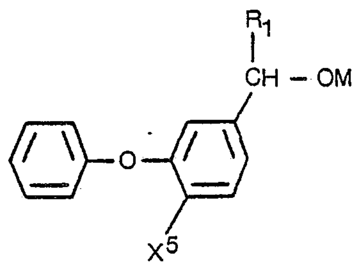

(I)

wherein M is

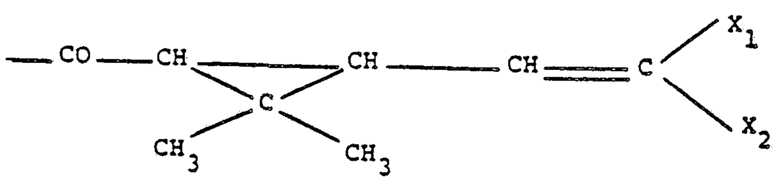

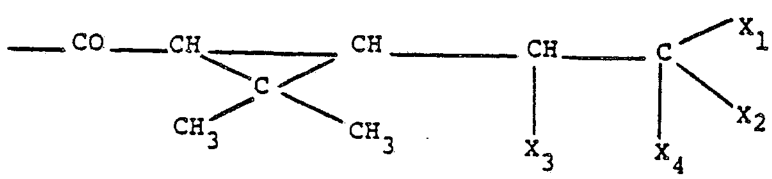

Or

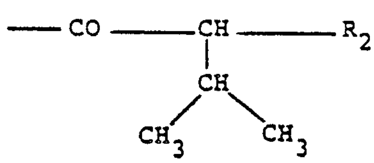

and wherein

$X_1$ to $X_4$ are independently selected from halo $C_1$—$C_4$ alkyl, halogen-substituted $C_1$—$C_4$ alkyl, and halogen-substituted phenyl;

$X_5$ is —H or halo;

$R_1$ is —H or cyano; and

$R_5$ is halogen-substituted phenyl.

10. Pour-on formulation according to claim 9 wherein the pyrethroid is selected from permethrin, phenothrin, deltamethrin, cypermethrin, cyhalothrin, flumethrin, cyfluthrin, cyphenothrin, tralomethrin, tralocythrin, fenvalerate or Fastac.

11. Pour-on formulation according to claim 10 wherein the pyrethroid is deltamethrin.

12. Pour-on formulation according to claim 8 wherein the ectoparasiticide is a wâter insoluble organophosphorous compound selected from bromophos-ethyl chlorfenvinphos, chlorpyrifos, coumaphos, Diazinon®, dichlofenthion, dioxathion, oxinothiophos, ethion, fenchlorphos, famphur, fenitrothion, phoxim and propetamphos.

13. Pour-on formulation according to claim 8 wherein the ectoparasiticide is amitraz.

14. A method of controlling external parasites which comprises making a localised external application of a formulation as defined in claim 1 herein to an animal.

15. A method according to claim 14 wherein the animal is selected from cattle, goats, pigs, horses, deer, sheep, cats and dogs.

16. A method according to either claim 14 or 15 wherein the formulation is used to control lice and keds on sheep or goats, or lice, flies or ticks on cattle.

17. A method of preparing a formulation as defined in claim 1 by standard techniques.

**Patentansprüche**

1. Übergießbare Formulierung enthaltend eines oder mehrere ectoparasitische Mittel in einem Glykol- oder Glycerolester einer $C_{8-10}$ Fettsaüre als Träger und bis zu 10% (W/V) üblicherweise in Formulierungen zum Übergießen verwendete Zusatzmittel.

2. Übergießbare Formulierung nach Anspruch 1, worin der Träger ein Glykoldiester oder ein Glyceroltriester einer $C_{8-10}$ Fettsaüre ist.

3. Übergießbare Formulierung nach Anspruch 2, worin der Träger fraktioniertes Kokosnußöl ist.

4. Übergießbare Formulierung nach einem der Ansprüche 1 bis 3, worin das ectoparasitische Mittel in Lösung vorliegt.

5. Übergießbare Formulierung nach einem der Ansprüche 1 bis 4, die ein Spreitungsmittel enthält.

6. Übergießbare Formulierung nach einem der Ansprüche 1 bis 5, die weniger als 1% (W/V) Zusatzmittel enthält.

7. Übergießbare Formulierung nach Anspruch 6, die einen oder mehrere Stabilisatoren und/oder Farbstoffe enthält.

8. Übergießbare Formulierung nach Anspruch 1, worin das ectoparasitische Mittel aus Pyrethrinen, Pyrethoiden, wasserunlöslichen Organophosphorverbindungen, Formamidinen, Avermectinen und Mischungen davon ausgewählt ist.

9. Formulierung nach Anspruch 8, worin das ectoparasitische Mittel ein Pyrethroid der Formel (I) ist

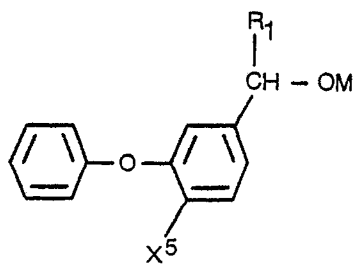

(I)

worin M

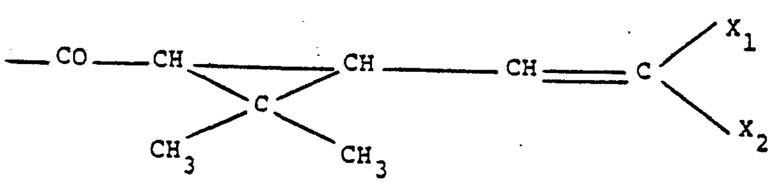

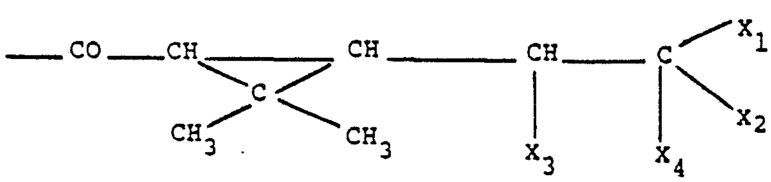

oder

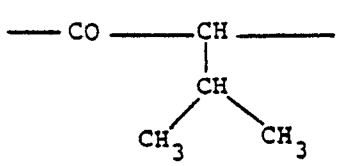

bedeutet, und worin

$X_1$ bis $X_4$ unabhängig voneinander aus Halogen, $C_1$—$C_4$-Alkyl halogensubstituiertem $C_1$—$C_4$-Alkyl und halogensubstituiertem Phenyl ausgewählt sind;

$X_5$ H oder Halogen bedeutet;

$R_1$ H oder Cyano bedeutet; und

$R_5$ halogensubstituiertes Phenyl bedeutet.

10. Übergießbare Formulierung nach Anspruch 9, worin das Pyrethroid aus Permethrin, Phenothrin, Deltamethrin, Cypermethrin, Cyhalothyrin, Flumethrin, Cyfluthrin, Cyphenothrin, Tralomethrin, Tralocythrin, Fenvalerat oder Fastac ausgewählt ist.

11. Übergießbare Formulierung nach Anspruch 10, worin das Pyrethroid Deltamethrin ist.

12. Übergießbare Formulierung nach Anspruch 8, worin das ectoparasitische Mittel eine wasserunlösliche Organophosphorverbindung ausgewählt aus Bromophos-ethyl, Chlorfenvinphos, Chlorpyrifos, Coumaphos, Diazinon®, Dichlofenthion, Dioxathion, Oxinothiophos, Ethion, Fenchlorphos, Famphur, Fenitrothion, Phoxim und Propetamphos ist.

13. Übergießbare Formulierung nach Anspruch 8, worin das ectoparasitische Mittel Amitras ist.

14. Verfahren zur Bekämpfung von externen Parasiten bei dem man eine Formulierung nach Anspruch 1 auf ein Tier extern lokalisiert appliziert.

15. Verfahren nach Anspruch 14, bei dem das Tier aus Rindern, Ziegen, Schweinen, Rotwild, Schafen, Katzen und Hunden ausgewält ist.

16. Verfahren nach Anspruch 14 oder 15, bei dem man die Formulierung zur Bekämpfung von Läusen und Zecken auf Schafen oder Ziegen, oder Läusen, Fliegen oder Zecken auf Rindern verwendet.

17. Verfahren zur Herstellung einer Formulierung nach Anspruch 1 durch Standardverfahren.

**Revendications**

1. Pour-on formulations comprenant un ou plusieurs ectoparasiticides dans un excipient qui est un ester du glycol ou du glycérol et d'un acide gras en $C_{8-10}$ et jusqu'à 10% p/v d'additifs d'usage classique dans les pour-on formulations.

2. Pour-on formulation suivant la revendication 1, dans laquelle l'excipient est un diester du glycol ou un triester du glycérol et d'un acide gras en $C_{8-10}$.

3. Pour-on formulation suivant la revendication 2, dans laquelle l'excipient est l'huile de coprah fractionnée.

4. Pour-on formulation suivant l'une quelconque des revendications 1 à 3, dans laquelle l'ectoparasiticide est en solution.

5. Pour-on formulation suivant l'une quelconque des revendications 1 à 4, qui contient un agent d'étalement.

6. Pour-on formulation suivant l'une quelconque des revendications 1 à 5, qui contient moins de 1% en volume d'additifs.

7. Pour-on formulation suivant la revendication 6, qui contient un ou plusieurs stabilisants et/ou agents colorants.

8. Pour-on formulation suivant la revendication 1, dans laquelle l'ectoparasiticide est choisi parmi les pyréthrines, les pyréthroîdes, les composés organophosphorés insolubles dans l'eau, les formamidines, les avermectines et leurs mélanges.

9. Pour-on formulation suivant la revendication 8, dans laquelle l'ectoparasiticide est un pyréthroîde de formule (I):

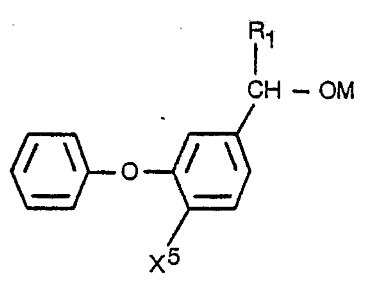

(I)

où M représente

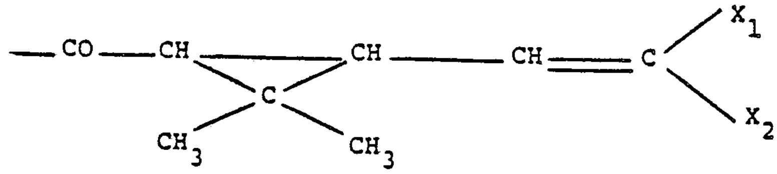

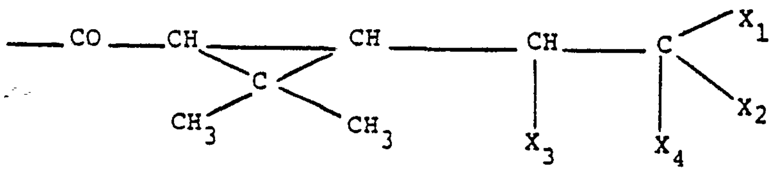

ou

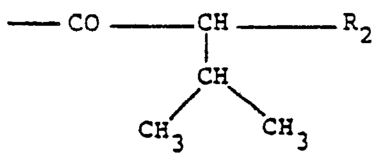

et où

$X_1$ à $X_4$ sont choisis indépendamment parmi halo, $C_{1-4}$-alcoyle, $C_{1-4}$-alcoyle halo-substitué et phényle halo-substitué;

$X_5$ représente H ou halo;

$R_1$ représente H ou cyano, et

$R_5$ représente phényle halo-substitué.

10. Pour-on formulation suivant la revendication 9, dans laquelle le pyréthroîde est choisi parmi la perméthrine, la phénothrine, la deltaméthrine, la cyperméthrine, la cyhalothrine, la fluméthrine, la cyfluthrine, la cyphénothrine, la tralométhrine, la tralocythrine, le fenvalérate et le Fastac.

11. Pour-on formulation suivant la revendication 10, dans laquelle le pyréthroîde est la deltaméthrine.

12. Pour-on formulation suivant la revendication 8, dans laquelle l'ectoparasiticide est un composé organophosporé insoluble dans l'eau choisi parmi le bromophos-éthyl, le chlorfenvinphos, le chlorpyrifos, le coumaphos, le Diazinon®, le dichlofenthion, la dioxathion, l'oxinothiophos, l'éthion, le fenchlorphos, le famphur, le fénitrithion, le phoxim et le propétamphos.

13. Pour-on formulation suivant la revendication 8, dans laquelle l'ectoparasiticide est l'amitraz.

14. Procédé pour lutter contre les parasites externes, qui comprend la pratique d'une application externe localisée d'une formulation telle que définie dans la revendication 1 sur un animal.

15. Procédé suivant la revendication 14, dans lequel l'animal est choisi parmi les bovins, les chèvres, les porcs, les chevaux, les cerfs, les moutons, les chats et les chiens.

16. Procédé suivant la revendication 14 ou 15, dans lequel la formulation est utilisée pour combattre les poux et les mélophages sur les moutons ou les chèvres, ou bien les poux, les mouches ou les tiques sur les bovins.

17. Procédé de préparation d'une formulation telle que définie dans la revendication 1 suivant des techniques classiques.